# EUROPEAN PATENT APPLICATION

(11) **EP 4 796 915 A1**
(43) Date of publication of application: **26.08.2026**
(21) Application number: 26159994.8
(22) Date of filing: 23.02.2026
(51) Int. Cl.: G01N 17/02

(54) **CURRENT DENSITY MEASURING APPARATUS AND CORROSION MEASURING METHOD**

(30) Priority: 25.02.2025 JP 2025028316
(71) Applicant: SHIMADZU CORPORATION, Kyoto-shi, Kyoto 604-8511 (JP); National Institute of Maritime, Port and Aviation Technology, Mitaka-shi, Tokyo 181-0004 (JP); Tokyo University of Science, Tokyo 162-8601 (JP)
(72) Inventor: MAJIMA, Yatsuse, Kyoto-shi, Kyoto 604-8511 (JP); NISHIMURA, Naoki, Kyoto-shi, Kyoto 604-8511 (JP); TOMISAKA, Masahiro, Kyoto-shi, Kyoto 604-8511 (JP); FUJIMOTO, Shuhei, Mitaka-shi, Tokyo 181-0004 (JP); YAMAJI, Toru, Mitaka-shi, Tokyo 181-0004 (JP); KOIKE, Kentaro, Mitaka-shi, Tokyo 181-0004 (JP); HASHIMOTO, Nagate, Shinjuku-ku, Tokyo 162-8601 (JP)
(74) Representative: Müller-Boré & Partner Patentanwälte PartG mbB

(57) **Abstract**

A current density measuring apparatus (100) includes an underwater electric field sensor (1) including a pair of electrode portions (10) and a potential difference (V) measuring unit configured to measure a potential difference (V) between the pair of electrode portions (10) without contacting a test object (80) placed in water, a conductivity meter (2) configured to measure a conductivity (σ) in the water, and a controller (3) configured or programmed to acquire a current density (J) of a current (70, 71) flowing from the test object (80) into the water using the potential difference (V) measured by the underwater electric field sensor (1) and the conductivity (σ) measured by the conductivity meter (2).

## Description

### BACKGROUND OF THE INVENTION

### Field of the Invention

The present invention relates to a current density measuring apparatus and a corrosion measuring method. Description of the Background Art

Conventionally, a corrosion measuring method for a test object in water is known. Such a corrosion measuring method is disclosed in Japanese Patent Laid-Open No. 53-033691, for example.

Japanese Patent Laid-Open No. 53-033691 discloses a current measuring apparatus including a metal to be measured placed in seawater in a test tank, a counter electrode placed in seawater in the test tank, an AC power source connected to the metal to be measured and the counter electrode, and a DC ammeter. The DC ammeter is provided in series between the metal to be measured and the counter electrode. In a corrosion measuring method for this metal to be measured in seawater, the DC current value in the circuit is measured using this current measuring apparatus, and the corrosion level of the metal to be measured is determined based on the measured DC current value.

The corrosion measuring method disclosed in Japanese Patent Laid-Open No. 53-033691 requires measuring the DC current value by electrically connecting the metal to be measured placed in seawater in the test tank to the counter electrode. However, in actual seawater (in water), the metal to be measured has marine organisms attached to it. Therefore, scraping work is required to electrically connect the metal to be measured (test object) to the counter electrode, and thus the effort required for this scraping work becomes a burden for a user. Scraping refers to removing marine organisms attached to the metal to be measured by crushing or peeling the marine organisms off. Therefore, it is desired to reduce the effort required of the user when the corrosion of the test object in water is measured.

### SUMMARY OF THE INVENTION

The present invention is intended to solve the above problem. The present invention aims to provide a current density measuring apparatus and a corrosion measuring method each capable of reducing the effort required of a user when corrosion of a test object in water is measured.

A current density measuring apparatus comprising:
an underwater electric field sensor including a pair of electrode portions and a potential difference measuring unit configured to measure a potential difference between the pair of electrode portions without contacting a test object placed in water;
a conductivity meter configured to measure a conductivity in the water; and
a controller configured or programmed to acquire a current density of a current flowing from the test object into the water using the potential difference measured by the underwater electric field sensor and the conductivity measured by the conductivity meter.

A corrosion measuring method comprising:
acquiring a current density without contacting a test object placed in water using a potential difference between a pair of electrode portions; and
determining corrosion of the test object using an acquired current density or detecting corrosion of the test object using the acquired current density.

The current density refers to the amount of electricity (charge) that flows per unit area per unit time.

The current density measuring apparatus described above can acquire the current density of the current flowing from the test object into the water without contacting the test object, using the potential difference measured by the underwater electric field sensor without contacting the test object and the conductivity in the water measured by the conductivity meter. Therefore, the current density of the current flowing in the vicinity of the test object in the water can be acquired without directly connecting the underwater electric field sensor electrically to the test object, and thus the need for scraping work to directly connect the underwater electric field sensor electrically to the test object can be eliminated. Thus, it is possible to provide the current density measuring apparatus capable of reducing the effort required of a user when the corrosion of the test object in the water is measured.

Furthermore, in the corrosion measuring method described above, the corrosion of the test object can be detected using the current density acquired without contacting the test object. Therefore, when the corrosion of the test object in the water is measured, the current density of the current flowing in the vicinity of the test object in the water can be acquired without directly connecting electrically to the test object, and thus the need for scraping work for directly connecting electrically to the test object can be eliminated. Thus, it is possible to provide the corrosion measuring method capable of reducing the effort required of a user when the corrosion of the test object in the water is measured.

The foregoing and other objects, features, aspects and advantages of the present invention will become more apparent from the following detailed description of the present invention when taken in conjunction with the accompanying drawings.

### BRIEF DESCRIPTION OF THE DRAWINGS

FIG. 1 is a block diagram showing the overall configuration of a current density measuring apparatus.
FIG. 2A is a sectional view of a pair of electrode portions, and FIG. 2B is a schematic view showing the pair of electrode portions and a pair of conductivity measuring electrodes as viewed from one side.
FIG. 3 is a schematic view for illustrating acquisition of current density.
FIG. 4 is a schematic view for illustrating a first example as a corrosion determination method for determining corrosion of a test object.
FIG. 5 is a schematic view for illustrating a second example as a corrosion detection method for detecting corrosion of a test object.
FIG. 6 is a diagram showing an example of a waveform of current density continuously acquired in the second example.

### DESCRIPTION OF THE PREFERRED EMBODIMENTS

An embodiment of the present invention is hereinafter described with reference to the drawings.

### Overall Configuration of Current Density Measuring Apparatus

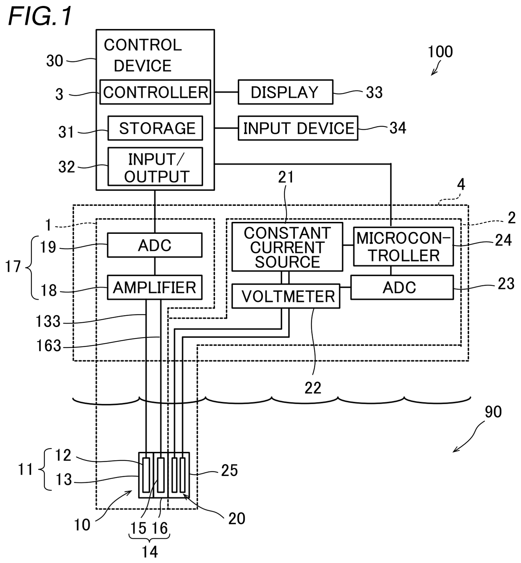

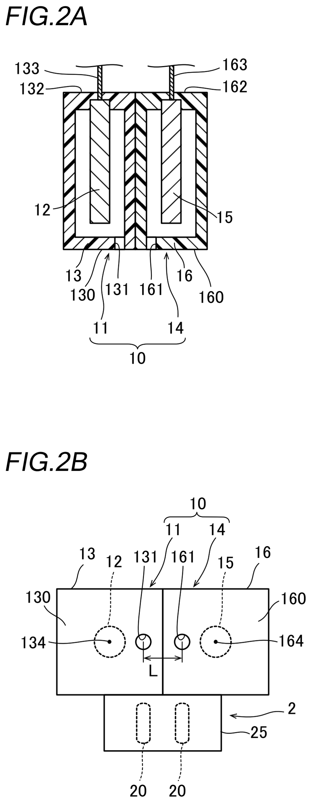

The overall configuration of a current density measuring apparatus 100 according to this embodiment is now described with reference to FIGS. 1, 2A, and 2B.

As shown in FIG. 1, the current density measuring apparatus 100 includes an underwater electric field sensor 1, a conductivity meter 2, and a controller 3.

The underwater electric field sensor 1 is used to measure an underwater electric potential (UEP). The underwater electric potential (potential difference V) refers to a minute potential difference in the sea 90 (in water). The underwater electric field sensor 1 includes a pair of electrode portions 10 and a potential difference measuring unit 17. The pair of electrode portions 10 include a first measuring electrode 12 and a second measuring electrode 15. The underwater electric field sensor 1 is configured to measure the potential difference V between the pair of electrode portions 10 without contacting a test object 80 in the sea 90. Specifically, the underwater electric field sensor 1 is configured to measure the potential difference V between the first measuring electrode 12 of a first electrode portion 11 and the second measuring electrode 15 of a second electrode portion 14 without contacting the test object 80 in the sea 90. A first housing 13 of the first electrode portion 11 and a second housing 16 of the second electrode portion 14 may contact marine organisms attached to the test object 80 in the sea 90 for measurement.

In this embodiment, the current density measuring apparatus 100 that measures the current density J "in the sea" and a corrosion measuring method that targets the test object 80 "in the sea" are described, but the present invention is not limited to "in the sea" and is not particularly limited as long as it is "in water". The term "in water" refers to "in seawater", "in lake water", "in river water", etc., for example. Also, the term "in water" refers to "in water stored in artificial structures such as pools, tanks, and aquariums. Furthermore, "water" of the term "in water" refers to seawater, freshwater, brackish water, etc., for example, and does not include pure water.

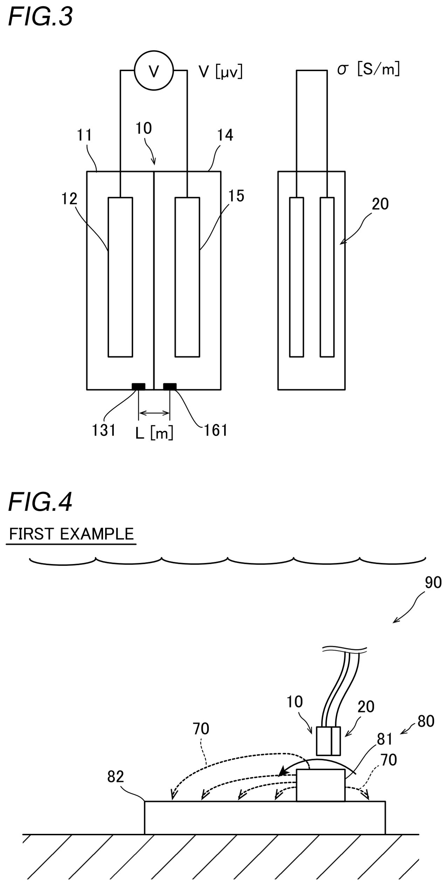

As shown in FIGS. 2A and 2B, the pair of electrode portions 10 include the first electrode portion 11 and the second electrode portion 14. The pair of electrode portions 10 are used in the sea 90 (see FIG. 4) in a state in contact with seawater. The pair of electrode portions 10 are used in the vicinity of the test object 80 (see FIG. 4) in the sea 90 without contacting the test object 80. The pair of electrode portions 10 may be disposed in a self-propelled device such as an underwater robot, an underwater drone, or an autonomous unmanned underwater vehicle, for example, or may be held by a diver.

The first electrode portion 11 includes the first measuring electrode 12 and the first housing 13. The first measuring electrode 12 is configured to measure a potential in the sea 90. As an example, the first measuring electrode 12 includes a silver-silver chloride electrode containing silver (Ag) and silver chloride (AgCl). The first measuring electrode 12 is connected to a first cable 133. The first measuring electrode 12 is connected to the potential difference measuring unit 17 (see FIG. 1) via the first cable 133.

The first measuring electrode 12 is disposed inside the first housing 13. The first measuring electrode 12 has a cylindrical shape and extends in the longitudinal direction of the first housing 13. The first measuring electrode 12 is aligned with the second measuring electrode 15 at a predetermined interval in the sea 90. The first measuring electrode 12 and the second measuring electrode 15 are disposed to maintain a constant interval therebetween.

The first housing 13 is configured to cover the first measuring electrode 12. The first housing 13 is made of an insulating material such as resin. The first housing 13 has a cylindrical shape, for example. Specifically, the first housing 13 has a rectangular cylindrical shape. A first opening 131 communicating with the outside is formed in a first end face 130 of the first housing 13 on the first side. The first opening 131 is configured to enable external seawater to flow into the first housing 13. When the current density measuring apparatus 100 is in use, the first measuring electrode 12 is immersed in seawater that flows into the first housing 13 through the first opening 131. The first cable 133 is inserted into a third end face 132 of the first housing 13 on the second side opposite to the first side.

The second electrode portion 14 includes the second measuring electrode 15 and the second housing 16. The second measuring electrode 15 is disposed inside the second housing 16. The second measuring electrode 15 is connected to the potential difference measuring unit 17 (see FIG. 1) via a second cable 163. The second housing 16 also has a rectangular cylindrical shape, for example. A second opening 161 communicating with the outside is formed in a second end face 160 of the second housing 16 on the first side. The second cable 163 is inserted into a fourth end face 162 of the second housing 16 on the second side opposite to the first side. The remaining configurations of the second electrode portion 14 are similar to those of the first electrode portion 11, and thus detailed description thereof is omitted.

The second electrode portion 14 is disposed adjacent to the first electrode portion 11. Specifically, the pair of electrode portions 10 are integrally provided such that the side surface of the first housing 13 of the first electrode portion 11 closer to the second electrode portion 14 contacts the side surface of the second housing 16 of the second electrode portion 14 closer to the first electrode portion 11. The pair of electrode portions 10 are integrally formed such that the first opening 131 of the first electrode portion 11 and the second opening 161 of the second electrode portion 14 are spaced a predetermined distance apart.

The pair of electrode portions 10 are integrally formed such that the predetermined distance is maintained between the first opening 131 and the second opening 161. The first opening 131 is provided closer to the second electrode portion 14 with respect to the center 134 of the first end face 130, and the second opening 161 is provided closer to the first electrode portion 11 with respect to the center 164 of the second end face 160. An opening-to-opening distance L (the center-to-center distance of the openings) between the first opening 131 and the second opening 161 is not particularly limited, and may be several millimeters, several centimeters, or 10 cm or more.

As shown in FIG. 1, the potential difference measuring unit 17 is configured to measure the potential difference V (underwater electric potential) between the first measuring electrode 12 of the first electrode portion 11 and the second measuring electrode 15 of the second electrode portion 14 without contacting the test object 80 (see FIG. 4) placed in water. In other words, the potential difference measuring unit 17 is configured to measure the potential difference V between the first measuring electrode 12 and the second measuring electrode 15 in a non-contact state with the test object 80.

The potential difference measuring unit 17 is housed inside a main housing 4. The main housing 4 is placed on land, on the sea, in the sea 90, inside a marine vessel, or in a self-propelled device such as an underwater robot, an underwater drone, or an autonomous unmanned underwater vehicle, for example. When the main housing 4 is placed on the sea or in the sea 90 and contacts seawater, the inside of the main housing 4 is sealed to prevent water from entering. The potential difference measuring unit 17 may be placed inside a housing other than the main housing 4.

The potential difference measuring unit 17 includes an amplifier 18 and an AD converter (ADC) 19. The amplifier 18 is configured to generate a signal obtained by amplifying the potential difference V between the first measuring electrode 12 and the second measuring electrode 15. One of a pair of input terminals of the amplifier 18 is connected to the first measuring electrode 12 via the first cable 133. The other of the pair of input terminals of the amplifier 18 is connected to the second measuring electrode 15 via the second cable 163.

The AD converter 19 is configured to convert the signal amplified by the amplifier 18 into a digital signal and output the converted digital signal to the controller 3. Thus, the potential difference measuring unit 17 outputs the potential difference V between the first measuring electrode 12 and the second measuring electrode 15 to the controller 3. The AD converter 19 is connected to the amplifier 18 and the controller 3.

The conductivity meter 2 is configured to measure the conductivity σ (electrical conductivity) in the sea 90. The conductivity meter 2 includes a pair of conductivity measuring electrodes 20, a constant current source 21, a voltmeter 22, an AD converter (ADC) 23, and a microcontroller 24. In other words, the conductivity meter 2 is an electrode type conductivity meter. The conductivity meter 2 may be an electromagnetic induction type conductivity meter. The constant current source 21, the voltmeter 22, the AD converter 23, and the microcontroller 24 are housed inside the main housing 4. The constant current source 21, the voltmeter 22, the AD converter 23, and the microcontroller 24 may be located inside a housing other than the main housing 4.

The pair of conductivity measuring electrodes 20 are used in the sea 90 in contact with seawater. The pair of conductivity measuring electrodes 20 are disposed inside a third housing 25. An opening (not shown) is formed in the third housing 25, and seawater flows into the third housing 25 through the opening. As shown in FIG. 2B, the third housing 25 is integrally formed with the first housing 13 of the first electrode portion 11 and the second housing 16 of the second electrode portion 14. That is, the pair of conductivity measuring electrodes 20 are disposed in the vicinity of the first electrode portion 11 and the second electrode portion 14. The pair of conductivity measuring electrodes 20 are used in the sea 90 in contact with seawater in the vicinity of the first electrode portion 11 and the second electrode portion 14. The pair of conductivity measuring electrodes 20 may be disposed together with the pair of electrode portions 10 in a self-propelled device such as an underwater robot, an underwater drone, or an autonomous unmanned underwater vehicle, for example, or may be held together with the pair of electrode portions 10 by a diver. Each of the pair of conductivity measuring electrodes 20 is connected to the voltmeter 22 via a cable.

As shown in FIG. 1, the constant current source 21 is a power supply configured to maintain an output current substantially constant regardless of the magnitude of the load (the resistance value between the pair of conductivity measuring electrodes 20). A pair of output terminals of the constant current source 21 are connected to the pair of conductivity measuring electrodes 20, respectively. The voltmeter 22 is connected between the pair of output terminals of the constant current source 21. The AD converter 23 is connected to the voltmeter 22 and the microcontroller 24.

The microcontroller 24 is connected to the constant current source 21, the AD converter 23, and the controller 3. The microcontroller 24 includes a processor such as a CPU (Central Processing Unit) that performs computational processing, and a memory that temporarily stores data during computations.

When the constant current source 21 outputs a current of a predetermined value, a current is generated through the seawater between the pair of conductivity measuring electrodes 20. The voltmeter 22 measures a potential difference between the pair of conductivity measuring electrodes 20 when a current is output from the constant current source 21. The AD converter 23 converts the output signal of the voltmeter 22 into a digital signal and outputs the converted digital signal to the microcontroller 24. The microcontroller 24 calculates the conductivity σ of the seawater between the pair of conductivity measuring electrodes 20 based on the output current value of the constant current source 21 and the potential difference obtained from the voltmeter 22. The microcontroller 24 outputs the calculated conductivity σ of the seawater between the pair of conductivity measuring electrodes 20 to the controller 3.

The controller 3 is configured to acquire the current density J of a current (70, 71) flowing from the test object 80 into the water using the potential difference V measured by the underwater electric field sensor 1 and the conductivity σ measured by the conductivity meter 2. The controller 3 includes a processor such as a CPU (Central Processing Unit) that performs computational processing, and a memory that temporarily stores data during computations.

The controller 3 is provided in a control device 30. The control device 30 includes a PC (personal computer), for example. The control device 30 includes the controller 3, a storage 31, and an input/output 32. The control device 30 is connected to a display 33 and an input device 34.

The storage 31 includes a volatile storage and a nonvolatile storage. The input/output 32 includes various interfaces for inputting and outputting signals to and from the control device 30. The input/output 32 is connected to the display 33 and the input device 34. The display 33 is a liquid crystal display, for example. The input device 34 includes a keyboard, a mouse, etc. The controller 3 acquires the potential difference V measured by the underwater electric field sensor 1 and the conductivity σ measured by the conductivity meter 2 via the input/output 32.

The control device 30 is placed on land, on the sea, inside a marine vessel, etc. The controller 3 may not be provided in the control device 30. The controller 3 may be housed inside the main housing 4, or may be placed inside a housing other than the main housing 4, for example.

### Acquisition of Current Density by Controller

Acquisition of the current density J by the controller 3 is now described with reference to FIG. 3. For the convenience of illustration, FIG. 3 illustrates the pair of electrode portions 10 in the underwater electric field sensor 1 and the conductivity meter 2 in parallel, but this is different from the actual arrangement of the underwater electric field sensor 1 and the conductivity meter 2.

The controller 3 acquires the potential difference V [µV] between the first measuring electrode 12 and the second measuring electrode 15 output from the underwater electric field sensor 1. Furthermore, the pair of electrode portions 10 are integrally formed such that the predetermined distance is maintained between the first opening 131 and the second opening 161, and thus the opening-to-opening distance L [m] (the center-to-center distance of the openings) between the first opening 131 and the second opening 161 is constant. The controller 3 acquires the opening-to-opening distance L, which is stored in advance in the storage 31, from the storage 31.

The controller 3 also acquires the conductivity σ [S/m] of the seawater between the pair of conductivity measuring electrodes 20 output from the conductivity meter 2. Because the pair of conductivity measuring electrodes 20 are disposed in the vicinity of the pair of electrode portions 10, the acquired conductivity σ can be estimated to be equivalent to the conductivity of the seawater at the position at which the potential difference V between the first measuring electrode 12 and the second measuring electrode 15 is measured.

The controller 3 then calculates the current density J [µA/m²] at the position at which the potential difference V between the first measuring electrode 12 and the second measuring electrode 15 is measured, using the following equation (1):$J \left[μA/{m}^{2}\right] = V \left[μV\right] / L \left[m\right] \times σ \left[S/m\right]$ ... (1) where J represents the current density at the position at which the potential difference V between the first measuring electrode 12 and the second measuring electrode 15 is measured, V represents the potential difference between the first measuring electrode 12 and the second measuring electrode 15, L represents the opening-to-opening distance between the first opening 131 and the second opening 161, and σ represents the conductivity of the seawater between the pair of conductivity measuring electrodes 20.

The controller 3 stores the calculated current density J in the storage 31. The controller 3 may store, in the storage 31, the calculated current density J in association with the position of the test object 80 corresponding to the position at which the potential difference V between the first measuring electrode 12 and the second measuring electrode 15 is measured. The controller 3 may also calculate the real-time current density J at the time of output of the potential difference V from the underwater electric field sensor 1 and at the time of output of the conductivity σ from the conductivity meter 2, using the potential difference V between the first measuring electrode 12 and the second measuring electrode 15 at the time of output from the underwater electric field sensor 1 and the conductivity σ of the seawater between the pair of conductivity measuring electrodes 20 at the time of output from the conductivity meter 2, and may display the calculated current density J in real time on the display 33.

### Corrosion determination Method for Test Object Using Current Density Measuring Apparatus

A first example of the corrosion measuring method for the test object 80 using the current density measuring apparatus 100 is now described with reference to FIG. 4. The first example of the corrosion measuring method for the test object 80 is a corrosion determination method for determining corrosion of the test object 80 using the current density J acquired by the current density measuring apparatus 100. The test object 80 is a sacrificial anode 81 located in the sea 90, for example. The sacrificial anode 81 is installed and electrically connected to an undersea structure 82 made of a steel material, for example.

As shown in FIG. 4, the undersea structure 82 is placed on the seabed. The undersea structure 82 is made of a steel material, with iron (Fe) as its main component, for example. The sacrificial anode 81 is installed in contact with the undersea structure 82. The sacrificial anode 81 is made of zinc (Zn), for example.

The materials of the sacrificial anode 81 and the undersea structure 82 are not limited to the above examples, and the material of the sacrificial anode 81 may be any material as long as the same has a higher ionization tendency than the material of the undersea structure 82. For example, the undersea structure 82 may be made of a steel material containing iron (Fe) as its main component, and the sacrificial anode 81 may be made of aluminum (Al), which has a higher ionization tendency than iron (Fe). Furthermore, the undersea structure 82 may not be placed on the seabed, but may be installed between the seabed and the sea surface.

The sacrificial anode 81 made of zinc (Zn) has a higher ionization tendency than the undersea structure 82 made of a steel material, with iron (Fe) as its main component. In other words, the sacrificial anode 81 made of zinc (Zn) is more likely to ionize than the undersea structure 82 made of a steel material. Therefore, the sacrificial anode 81 oxidizes (dissolves and corrodes) while supplying a corrosion protection current 70 to the undersea structure 82. Consequently, corrosion of the undersea structure 82 is reduced or prevented.

However, as oxidation (dissolution, corrosion) of the sacrificial anode 81 progresses, the corrosion protection current 70 supplied from the sacrificial anode 81 decreases. Thus, it is difficult to reduce or prevent corrosion of the undersea structure 82. Therefore, the current density J acquired by the current density measuring apparatus 100 is used to determine the corrosion (deterioration) of the sacrificial anode 81.

Determining the corrosion of the sacrificial anode 81 using the current density J acquired by the current density measuring apparatus 100 includes a step of acquiring the current density J without contacting the sacrificial anode 81 placed in the sea 90 using the potential difference V between the pair of electrode portions 10 of the underwater electric field sensor 1, and a step of determining the corrosion of the sacrificial anode 81 using the acquired current density J.

The step of acquiring the current density J includes a step of measuring the potential difference V between the pair of electrode portions 10 without contacting the sacrificial anode 81, a step of measuring the conductivity σ in the sea 90, and a step of acquiring the current density J of the current 70 (corrosion current) flowing from the sacrificial anode 81 into the sea 90 using the measured potential difference V and the measured conductivity σ.

The step of measuring the potential difference V between the pair of electrode portions 10 without contacting the sacrificial anode 81 is performed by using the pair of electrode portions 10 of the underwater electric field sensor 1 to measure the potential difference V without contacting the sacrificial anode 81. Specifically, the pair of electrode portions 10 of the underwater electric field sensor 1 are disposed on a self-propelled undersea device such as an underwater robot, an underwater drone, or an autonomous unmanned underwater vehicle, for example, or are held by a diver. The underwater device or the diver moves in the vicinity of the sacrificial anode 81 and moves the pair of electrode portions 10 along the vicinity of the surface of the sacrificial anode 81. The potential difference measuring unit 17 measures the potential difference V between the first measuring electrode 12 and the second measuring electrode 15, which are in a non-contact state with the sacrificial anode 81. The potential difference measuring unit 17 outputs the potential difference V between the first measuring electrode 12 and the second measuring electrode 15 to the controller 3.

The step of measuring the conductivity σ in the sea 90 is performed by using the pair of conductivity measuring electrodes 20 of the conductivity meter 2, which are integrally provided with the pair of electrode portions 10 of the underwater electric field sensor 1, to calculate the conductivity σ of the seawater. Specifically, similarly to the pair of electrode portions 10 of the underwater electric field sensor 1, the pair of conductivity measuring electrodes 20 are disposed in a self-propelled undersea device or the like, or held by a diver. The underwater device or the diver moves in the vicinity of the sacrificial anode 81 and moves the pair of conductivity measuring electrodes 20 along the vicinity of the surface of the sacrificial anode 81. The microcontroller 24 of the conductivity meter 2 calculates the conductivity σ of the seawater between the pair of conductivity measuring electrodes 20 based on the output current value of the constant current source 21 and the potential difference V obtained from the voltmeter 22. The microcontroller 24 outputs the calculated conductivity σ of the seawater between the pair of conductivity measuring electrodes 20 to the controller 3.

The step of acquiring the current density J of the current 70 flowing from the sacrificial anode 81 into the sea 90 is performed by the controller 3 using the potential difference V measured by the underwater electric field sensor 1 and the conductivity σ measured by the conductivity meter 2. The controller 3 acquires the current density J by calculating the current density J at the position at which the potential difference V between the first measuring electrode 12 and the second measuring electrode 15 is measured based on the above equation (1). The controller 3 also stores the acquired current density J in the storage 31.

The current density J to be acquired may be only the current density J at one position at which the potential difference V between the first measuring electrode 12 and the second measuring electrode 15 is measured, or may be a plurality of current densities J at a plurality of positions at which the potential difference V between the first measuring electrode 12 and the second measuring electrode 15 is measured. In estimating the sum of the currents 70 flowing from the sacrificial anode 81 described below, it is preferable to acquire a plurality of current densities J at a plurality of positions because estimating the sum of the currents 70 using a plurality of current densities J is closer to the true value of the currents 70 flowing from the sacrificial anode 81 than estimating the sum of the currents 70 using only one current density J.

The step of determining the corrosion of the sacrificial anode 81 using the acquired current density J includes a step of estimating the sum of the currents 70 flowing from the sacrificial anode 81 by integrating the acquired current densities J, and a step of determining the corrosion of the sacrificial anode 81 using the estimated sum of the currents 70. The step of determining the corrosion of the sacrificial anode 81 using the acquired current density J may be performed substantially simultaneously with the step of acquiring the current density J, or may be performed after the step of acquiring the current density J.

The step of estimating the sum of the currents 70 flowing from the sacrificial anode 81 is performed by the controller 3 integrating the acquired current densities J. Specifically, the step of estimating the sum of the currents 70 flowing from the sacrificial anode 81 is performed by the controller 3 integrating the acquired current densities J over the area of the sacrificial anode 81. As described above, the current density J refers to the amount of electricity (charge) that flows per unit area per unit time. The acquired current densities J are integrated over the area of the sacrificial anode 81 such that the sum of the currents 70 flowing from the sacrificial anode 81 can be estimated. The step of estimating the sum of the currents 70 flowing from the sacrificial anode 81 may be performed by the controller 3 adding up the plurality of acquired current densities J.

For example, the step of determining the corrosion of the sacrificial anode 81 is performed by determining that the sacrificial anode 81 is corroded when the estimated sum of the currents 70 is equal to or less than a predetermined threshold. As oxidation (dissolution, corrosion) of the sacrificial anode 81 progresses, the current 70 (corrosion protection current) supplied from the sacrificial anode 81 decreases, and the function of the sacrificial anode 81 decreases. The current 70 (corrosion protection current) supplied from the sacrificial anode 81 can be considered equivalent to the estimated sum of the currents 70 flowing from the sacrificial anode 81. Therefore, when the estimated sum of the currents 70 is equal to or less than the predetermined threshold, the sacrificial anode 81 is determined to be corroded. Furthermore, when the estimated sum of the currents 70 exceeds the predetermined threshold, the sacrificial anode 81 is determined not to be corroded. The predetermined threshold is not particularly limited and may be 90%, less than 90%, or more than 90% of the estimated sum of the currents 70 at the time at which the sacrificial anode 81 is installed in the sea 90.

The determination of corrosion of the sacrificial anode 81 using the estimated sum of the currents 70 may be performed by the controller 3 or by a user. When the determination of corrosion of the sacrificial anode 81 is performed by the controller 3, the controller 3 estimates the sum of the currents 70 flowing from the sacrificial anode 81 by integrating the current densities J stored in the storage 31 over the area of the sacrificial anode 81 stored in advance in the storage 31, for example. The controller 3 determines that the sacrificial anode 81 is corroded when the estimated sum of the currents 70 is equal to or less than the predetermined threshold, and determines that the sacrificial anode 81 is not corroded when the estimated sum of the currents 70 exceeds the predetermined threshold.

When the determination of corrosion of the sacrificial anode 81 is performed by the user, the controller 3 estimates the sum of the currents 70 flowing from the sacrificial anode 81 by integrating the current densities J stored in the storage 31 over the area of the sacrificial anode 81 stored in advance in the storage 31, and displays the estimated sum of the currents 70 on the display 33, for example. When the sum of the currents 70 estimated by the controller 3 and displayed on the display 33 is equal to or less than the predetermined threshold, the user determines that the sacrificial anode 81 is corroded, and when the estimated sum of the currents 70 exceeds the predetermined threshold, the user determines that the sacrificial anode 81 is not corroded.

### Corrosion Detection Method for Test Object Using Current Density Measuring Apparatus

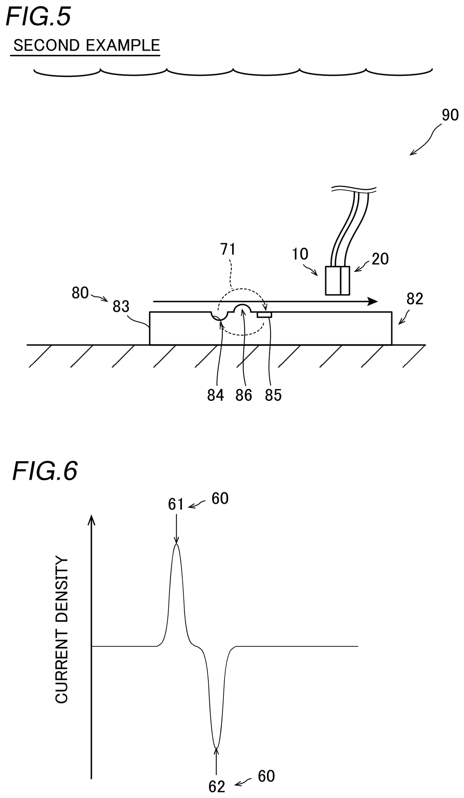

Next, a second example of the corrosion measuring method for the test object 80 using the current density measuring apparatus 100 is described with reference to FIG. 5. The second example of the corrosion measuring method for the test object 80 is a corrosion detection method for detecting corrosion of the test object 80 using the current density J acquired by the current density measuring apparatus 100. The test object 80 is an undersea structure 82, for example. The undersea structure 82 is made of a non-corrosion-protected steel material 83, for example. The non-corrosion-protected steel material 83 is a steel material that has not been subjected to corrosion protection treatment.

As shown in FIG. 5, the undersea structure 82 is placed on the seabed. The undersea structure 82 is made of the non-corrosion-protected steel material 83, with iron (Fe) as its main component, for example. The material of the undersea structure 82 is not limited to the above example, and may be aluminum, for example. Furthermore, the undersea structure 82 may not be placed on the seabed, but may be installed between the seabed and the sea surface.

Over time, the non-corrosion-protected steel material 83 placed in the sea 90 develops a depression (thinned portion) on the steel material surface due to corrosion, and a protrusion 86 formed by accumulating rust in the vicinity of the depression due to corrosion. Specifically, over time, the non-corrosion-protected steel material 83 placed in the sea 90 develops an anode portion 84 and a cathode portion 85 with different potential differences V on the steel material surface. The anode portion 84 is a depression (thinned portion) due to corrosion. The cathode portion 85 can be a portion in the vicinity of the anode portion 84.

As iron (Fe) oxidizes, a current 71 flows from the anode portion 84 to the cathode portion 85 inside the steel material, and the current 71 that has flowed to the anode portion 84 flows through seawater and returns to the cathode portion 85. In other words, the current 71 flows between the anode portion 84 and the cathode portion 85 formed on the non-corrosion-protected steel material 83 placed in the sea 90.

The corrosion reaction can be expressed by the following formula.
anode portion 84: Fe → Fe²⁺ + 2e⁻
cathode portion 85: 1/2O₂ + H₂O + 2e⁻ → 2OH⁻

The corrosion reaction of the steel material is the sum of the anodic and cathodic reactions, and thus ferrous hydroxide (Fe(OH)₂) is produced.

Fe + 1/2O₂ + H₂O → Fe²⁺ + 2OH⁻ → Fe(OH)₂

Because this ferrous hydroxide is unstable, it is further oxidized to ferric hydroxide or ferric oxide (red rust) and deposited such that the steel material undergoes thinning, and a rust layer is formed.

When the corroded depression (thinned portion) is covered with marine organisms or the like, it is difficult to detect the corroded depression through a visual inspection by a diver or an inspection by visually checking an image captured by a camera mounted on a self-propelled undersea device. Therefore, the current density J acquired by the current density measuring apparatus 100 is used to detect corrosion of the non-corrosion-protected steel material 83.

Detection of the corrosion of the non-corrosion-protected steel material 83 using the current density J acquired by the current density measuring apparatus 100 includes a step of acquiring the current density J in a non-contact manner with respect to the non-corrosion-protected steel material 83 placed in the sea 90 using the potential difference V between the pair of electrode portions 10 of the underwater electric field sensor 1, and a step of detecting the corrosion of the non-corrosion-protected steel material 83 using the acquired current density J. The step of acquiring the current density J is similar to the step of acquiring the current density J in the corrosion determination method for the test object 80 described above, and thus description thereof is omitted.

The step of acquiring the current density J is performed by screening the surface of the non-corrosion-protected steel material 83 using the current density measuring apparatus 100. That is, an underwater device or a diver moves the pair of electrode portions 10 and the pair of conductivity measuring electrodes 20 along the vicinity of the surface of the non-corrosion-protected steel material 83 to continuously acquire the current density J in the vicinity of the surface of the non-corrosion-protected steel material 83.

The step of detecting the corrosion of the non-corrosion-protected steel material 83 using the acquired current density J is performed by detecting the corrosion of the non-corrosion-protected steel material 83 using a peak portion 60 (see FIG. 6) of the waveform of the acquired current density J. That is, the step of detecting the corrosion of the non-corrosion-protected steel material 83 using the acquired current density J is performed by detecting that the non-corrosion-protected steel material 83 is corroded when there is the peak portion 60 in the waveform of the continuously acquired current density J, based on the current 71 flowing between the anode portion 84 and the cathode portion 85 formed on the non-corrosion-protected steel material 83 placed in the sea 90. The step of detecting the corrosion of the non-corrosion-protected steel material 83 using the acquired current density J may be performed substantially simultaneously with the step of acquiring the current density J, or may be performed after the step of acquiring the current density J.

The distance between the anode portion 84 and the cathode portion 85 formed on the non-corrosion-protected steel material 83 is extremely small, and the current 71 flows across this minute gap. Therefore, the opening-to-opening distance L (the center-to-center distance of the openings) between the first opening 131 and the second opening 161 shown in FIG. 3 is preferably smaller. The opening-to-opening distance L between the first opening 131 and the second opening 161 is reduced such that the spatial resolution of the underwater potential (potential difference V) can be improved.

As shown in FIG. 6, the peak portion 60 of the waveform of the acquired current density J is detected by extracting a peak portion 61 of the crest and a peak portion 62 of the valley from the generated waveform of the current density J. When the anode portion 84 and the cathode portion 85 are formed on the non-corrosion-protected steel material 83, the waveform of the continuously acquired current density J has the peak portion 61 of the crest and the peak portion 62 of the valley. The peak portion 61 of the crest in the waveform of the current density J indicates the anode portion 84. That is, the peak portion 61 of the crest in the waveform of the current density indicates a corroded depression (thinned portion). Furthermore, the peak portion 62 of the valley in the waveform of the current density J indicates the cathode portion 85. Therefore, when the peak portion 60 is included in the waveform of the continuously acquired current density J, it indicates that the non-corrosion-protected steel material 83 is corroded.

In the step of detecting the corrosion of the non-corrosion-protected steel material 83, the controller 3 generates the waveform of the continuously acquired current density J. Furthermore, the extraction of the peak portion 60 in the generated waveform of the current density J may be performed by the controller 3 or by the user. When the controller 3 detects the corrosion of the non-corrosion-protected steel material 83, the controller 3 generates the waveform of the continuously acquired current density J and determines whether or not the waveform of the current density J has the peak portion 60, for example. The controller 3 detects the corrosion of the non-corrosion-protected steel material 83 when the waveform of the current density J has the peak portion 60, and does not detect the corrosion of the non-corrosion-protected steel material 83 when the waveform of the current density J does not have the peak portion 60.

When the detection of the corrosion of the non-corrosion-protected steel material 83 is performed by the user, the controller 3 generates the waveform of the continuously acquired current density J and displays the generated waveform of the current density J on the display 33, for example. The user then determines whether or not the generated waveform of the current density J has the peak portion 60, for example. When the waveform of the current density J has the peak portion 60, the user detects the corrosion of the non-corrosion-protected steel material 83, and when the waveform of the current density J does not have the peak portion 60, the user does not detect the corrosion of the non-corrosion-protected steel material 83.

The current density J in the vicinity of the surface of the non-corrosion-protected steel material 83 is continuously acquired by an underwater device or a diver moving the pair of electrode portions 10 and the pair of conductivity measuring electrodes 20 along the vicinity of the surface of the non-corrosion-protected steel material 83. Therefore, when the measurement time and measurement route using the pair of electrode portions 10 and the pair of conductivity measuring electrodes 20 with respect to the non-corrosion-protected steel material 83 are known, it can be said that the horizontal axis of the waveform of the current density J indicates the measurement time resulting from movement along the vicinity of the surface of the non-corrosion-protected steel material 83, and also indicates the measurement position in the vicinity of the surface of the non-corrosion-protected steel material 83. Therefore, the controller 3 may be configured to detect the location of corrosion in the non-corrosion-protected steel material 83 based on the position of the peak portion 60 in the waveform of the current density J, or the user may detect the location of corrosion in the non-corrosion-protected steel material 83 based on the position of the peak portion 60 in the waveform of the current density J.

### Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

For example, the controller or the user may determine corrosion of a metal other than the sacrificial anode as the test object using the acquired current density. For example, the controller or the user may determine corrosion of a metal coating the undersea structure using the acquired current density.

Furthermore, for example, the controller or the user may detect corrosion of a metal other than the non-corrosion-protected steel material as the test object using the acquired current density. For example, the controller or the user may detect corrosion of stainless steel in the sea using the acquired current density.

Furthermore, for example, the pair of electrode portions may include a plurality of pairs of electrode portions. That is, the pair of electrode portions may include a first measuring electrode, a second measuring electrode, and a third measuring electrode, the first measuring electrode may be shared, a first pair of electrode portions may measure a potential difference between the first measuring electrode and the second measuring electrode, and a second pair of electrode portions may measure a potential difference between the first measuring electrode and the third measuring electrode. In such a case, the third measuring electrode may be disposed in a direction perpendicular to a direction in which the second measuring electrode is located relative to the first measuring electrode.

Furthermore, for example, the pair of electrode portions may not be integrally formed. That is, the pair of electrode portions may not be integrally formed with the side surface of the first housing of the first electrode portion closer to the second electrode portion being in contact with the side surface of the second housing of the second electrode portion closer to the first electrode portion, but the pair of electrode portions may be provided separately and individually. When the pair of electrode portions are integrally formed, a diver or the like can easily hold the pair of integrally formed electrode portions with one hand, and thus convenience (usability) can be improved. Furthermore, for example, the pair of electrode portions may be configured such that the opening-to-opening distance between the respective openings provided in the pair of electrode portions is variable.

Furthermore, for example, the first opening may be provided at the center of the first end face or in a portion of the first end face farther away from the second electrode portion, and the second opening may be provided at the center of the second end face or in a portion of the second end face farther away from the first electrode portion.

Furthermore, for example, in the determination of the corrosion of the sacrificial anode, the current densities may be used to estimate the sum of the currents flowing from the sacrificial anode by another method so as to determine the corrosion of the sacrificial anode.

Furthermore, for example, in the detection of the corrosion of the non-corrosion-protected steel material, the corrosion of the sacrificial anode may be detected by another method using the current density, without using the peak portion of the waveform of the current density.

### Aspects

It will be appreciated by those skilled in the art that the exemplary embodiments described above are specific examples of the following aspects.

### (Item 1)

A current density measuring apparatus comprising:
an underwater electric field sensor including a pair of electrode portions and a potential difference measuring unit configured to measure a potential difference between the pair of electrode portions without contacting a test object placed in water;
a conductivity meter configured to measure a conductivity in the water; and
a controller configured or programmed to acquire a current density of a current flowing from the test object into the water using the potential difference measured by the underwater electric field sensor and the conductivity measured by the conductivity meter.

The current density of the current flowing from the test object into the water can be acquired without contacting the test object using the potential difference measured by the underwater electric field sensor without contacting the test object and the conductivity in the water measured by the conductivity meter. Therefore, the current density of the current flowing in the vicinity of the test object in the water can be acquired without directly connecting the underwater electric field sensor electrically to the test object, and thus the need for scraping work to directly connect the underwater electric field sensor electrically to the test object can be eliminated. Thus, it is possible to provide the current density measuring apparatus capable of reducing the effort required of a user when corrosion of the test object in the water is measured.

### (Item 2)

The current density measuring apparatus according to item 1, wherein the pair of electrode portions include respective openings, the openings being spaced a predetermined distance apart.

In this case, an opening-to-opening distance between the respective openings provided in the pair of electrode portions is a predetermined value, and thus the current density of the current flowing from the test object into the water can be easily acquired using the opening-to-opening distance, which is the predetermined value.

### (Item 3)

The current density measuring apparatus according to item 1 or 2, wherein the controller is configured or programmed to determine corrosion of the test object using an acquired current density or to detect corrosion of the test object using the acquired current density.

In this case, the corrosion of the test object can be determined or detected using the current density acquired without directly contacting the test object, and thus the effort required of the user to perform scraping work, for example, can be reduced when corrosion of the test object is determined or detected.

### (Item 4)

The current density measuring apparatus according to any one of items 1 to 3, wherein
the underwater electric field sensor is configured to measure the potential difference between the pair of electrode portions without contacting a sacrificial anode as the test object; and
the controller is configured or programmed to perform:
   a control to acquire a current density of a current flowing from the sacrificial anode into the water using the potential difference measured by the underwater electric field sensor and the conductivity measured by the conductivity meter; and
   a control to determine corrosion of the sacrificial anode using an acquired current density.

In this case, the current density of the current flowing into the water from the sacrificial anode as the test object is acquired such that the corrosion of the sacrificial anode, which oxidizes (dissolves) while supplying a corrosion protection current to an undersea structure, can be appropriately determined using the acquired current density. In addition, the controller determines the corrosion of the sacrificial anode using the acquired current density, and thus the corrosion of the sacrificial anode can be easily determined.

### (Item 5)

The current density measuring apparatus according to item 4, wherein the controller is configured or programmed to estimate a sum of currents flowing from the sacrificial anode by integrating acquired current densities, and determine corrosion of the sacrificial anode using an estimated sum of the currents.

In this case, the sum of the currents flowing from the sacrificial anode is estimated by integrating a plurality of acquired current densities, and the corrosion of the sacrificial anode, which oxidizes (dissolves) while supplying a corrosion protection current to the undersea structure, can be accurately determined using the estimated sum of the currents.

### (Item 6)

The current density measuring apparatus according to any one of items 1 to 3, wherein
the underwater electric field sensor is configured to measure the potential difference between the pair of electrode portions without contacting a non-corrosion-protected steel material as the test object; and
the controller is configured or programmed to perform:
   a control to acquire a current density of a current flowing from the non-corrosion-protected steel material into the water using the potential difference measured by the underwater electric field sensor and the conductivity measured by the conductivity meter; and
   a control to detect corrosion of the non-corrosion-protected steel material using an acquired current density.

In this case, the current density of the current flowing into the water from the non-corrosion-protected steel material that has not been subjected to corrosion protection treatment as the test object is acquired such that the non-corrosion-protected steel material that has corroded due to oxidation can be appropriately detected using the acquired current density. In addition, the controller detects the corrosion of the non-corrosion-protected steel material using the acquired current density, and thus the corrosion of the non-corrosion-protected steel material can be easily detected.

### (Item 7)

The current density measuring apparatus according to item 6, wherein the controller is configured or programmed to detect corrosion of the non-corrosion-protected steel material using a peak portion of a waveform of the acquired current density.

In this case, the non-corrosion-protected steel material that has corroded by oxidation can be easily detected using the peak portion of the waveform of the acquired current density.

### (Item 8)

The current density measuring apparatus according to item 2, wherein
the pair of electrode portions include:
   a first electrode portion including a first measuring electrode configured to measure a potential in the water, and a cylindrical first housing including, in a first end face, a first opening of the openings communicating with an outside and configured to cover the first measuring electrode; and
   a second electrode portion including a second measuring electrode configured to measure a potential in the water, and a cylindrical second housing including, in a second end face, a second opening of the openings communicating with the outside and configured to cover the second measuring electrode, the second electrode portion being disposed adjacent to the first electrode portion; and
the first opening is disposed closer to the second electrode portion with respect to a center of the first end face, and the second opening is disposed closer to the first electrode portion with respect to a center of the second end face.

In this case, the opening-to-opening distance between the first opening and the second opening can be reduced as compared with a case in which the first opening is provided at the center of the first end face and the second opening is provided at the center of the second end face, and thus the spatial resolution of the underwater potential (potential difference) can be improved. Therefore, the accuracy of determining or detecting the corrosion of the test object using the current density, for example, can be improved.

### (Item 9)

A corrosion measuring method comprising:
acquiring a current density without contacting a test object placed in water using a potential difference between a pair of electrode portions; and
determining corrosion of the test object using an acquired current density or detecting corrosion of the test object using the acquired current density.

The corrosion of the test object can be detected using the current density acquired without contacting the test object. Therefore, when the corrosion of the test object in the water is measured, the current density of the current flowing in the vicinity of the test object in the water can be acquired without directly connecting electrically to the test object, and thus the need for scraping work for directly connecting electrically to the test object can be eliminated. Thus, it is possible to provide the corrosion measuring method capable of reducing the effort required of a user when the corrosion of the test object in the water is measured.

### (Item 10)

The corrosion measuring method according to item 9, wherein
the acquiring of the current density includes:
measuring the potential difference between the pair of electrode portions without contacting the test object;
measuring a conductivity in the water; and
acquiring a current density of a current flowing from the test object into the water using a measured potential difference and a measured conductivity.

In this case, the current density of the current flowing from the test object into the water can be appropriately acquired without contacting the test object using the potential difference measured without contacting the test object and the measured conductivity in the water. Thus, the current density can be accurately acquired without contacting the test object.

### (Item 11)

The corrosion measuring method according to item 10, wherein
the measuring of the potential difference includes measuring the potential difference between the pair of electrode portions without contacting a sacrificial anode as the test object; and
the determining of corrosion of the test object or the detecting of corrosion of the test object includes determining corrosion of the sacrificial anode using the acquired current density.

In this case, the current density of the current flowing into the water from the sacrificial anode as the test object is acquired such that the corrosion of the sacrificial anode, which oxidizes (dissolves) while supplying a corrosion protection current to an undersea structure, can be appropriately determined using the acquired current density.

### (Item 12)

The corrosion measuring method according to item 11, wherein the determining of corrosion of the sacrificial anode includes estimating a sum of currents flowing from the sacrificial anode by integrating acquired current densities and determining corrosion of the sacrificial anode using an estimated sum of the currents.

In this case, the sum of the currents flowing from the sacrificial anode is estimated by integrating a plurality of acquired current densities, and the corrosion of the sacrificial anode, which oxidizes (dissolves) while supplying a corrosion protection current to the undersea structure, can be accurately determined using the estimated sum of the currents.

### (Item 13)

The corrosion measuring method according to item 10, wherein
the measuring of the potential difference includes measuring the potential difference between the pair of electrode portions without contacting a non-corrosion-protected steel material as the test object; and
the determining of corrosion of the test object or the detecting of corrosion of the test object includes detecting corrosion of the non-corrosion-protected steel material using the acquired current density.

In this case, the current density of the current flowing into the water from the non-corrosion-protected steel material that has not been subjected to corrosion protection treatment as the test object is acquired such that the non-corrosion-protected steel material that has corroded due to oxidation can be appropriately detected using the acquired current density.

### (Item 14)

The corrosion measuring method according to item 13, wherein the detecting of corrosion of the non-corrosion-protected steel material includes detecting corrosion of the non-corrosion-protected steel material using a peak portion of a waveform of the acquired current density.

In this case, the non-corrosion-protected steel material that has corroded by oxidation can be easily detected using the peak portion of the waveform of the acquired current density.

## Claims

1. A current density measuring apparatus (100) comprising:
an underwater electric field sensor (1) including a pair of electrode portions (10) and a potential difference measuring unit (17) configured to measure a potential difference (V) between the pair of electrode portions (10) without contacting a test object (80) placed in water;
a conductivity meter (2) configured to measure a conductivity (σ) in the water; and
a controller (3) configured or programmed to acquire a current density (J) of a current (70, 71) flowing from the test object (80) into the water using the potential difference (V) measured by the underwater electric field sensor (1) and the conductivity (σ) measured by the conductivity meter (2).

2. The current density measuring apparatus (100) according to claim 1, wherein the pair of electrode portions (10) include respective openings (131, 161), the openings (131, 161) being spaced a predetermined distance apart.

3. The current density measuring apparatus (100) according to claim 1, wherein the controller (3) is configured or programmed to determine corrosion of the test object (80) using an acquired current density (J) or to detect corrosion of the test object (80) using the acquired current density (J).

4. The current density measuring apparatus (100) according to claim 1, wherein
the underwater electric field sensor (1) is configured to measure the potential difference (V) between the pair of electrode portions (10) without contacting a sacrificial anode (81) as the test object (80); and
the controller (3) is configured or programmed to perform:
a control to acquire a current density (J) of a current (70, 71) flowing from the sacrificial anode (81) into the water using the potential difference (V) measured by the underwater electric field sensor (1) and the conductivity (σ) measured by the conductivity meter (2); and
a control to determine corrosion of the sacrificial anode (81) using an acquired current density (J).

5. The current density measuring apparatus (100) according to claim 4, wherein the controller (3) is configured or programmed to estimate a sum of currents flowing from the sacrificial anode (81) by integrating acquired current densities, and determine corrosion of the sacrificial anode (81) using an estimated sum of the currents.

6. The current density measuring apparatus (100) according to claim 1, wherein
the underwater electric field sensor (1) is configured to measure the potential difference (V) between the pair of electrode portions (10) without contacting a non-corrosion-protected steel material (83) as the test object (80); and
the controller (3) is configured or programmed to perform:
a control to acquire a current density (J) of a current (70, 71) flowing from the non-corrosion-protected steel material (83) into the water using the potential difference (V) measured by the underwater electric field sensor (1) and the conductivity (σ) measured by the conductivity meter (2); and
a control to detect corrosion of the non-corrosion-protected steel material (83) using an acquired current density (J).

7. The current density measuring apparatus (100) according to claim 6, wherein the controller (3) is configured or programmed to detect corrosion of the non-corrosion-protected steel material (83) using a peak portion (60) of a waveform of the acquired current density (J).

8. The current density measuring apparatus (100) according to claim 2, wherein
the pair of electrode portions (10) include:
a first electrode portion (11) including a first measuring electrode (12) configured to measure a potential in the water, and a cylindrical first housing (13) including, in a first end face (130), a first opening (131) of the openings (131, 161) communicating with an outside and configured to cover the first measuring electrode (12); and
a second electrode portion (14) including a second measuring electrode (15) configured to measure a potential in the water, and a cylindrical second housing (16) including, in a second end face (160), a second opening (161) of the openings (131, 161) communicating with the outside and configured to cover the second measuring electrode (15), the second electrode portion (14) being disposed adjacent to the first electrode portion (11); and
the first opening (131) is disposed closer to the second electrode portion (14) with respect to a center (134) of the first end face (130), and the second opening (161) is disposed closer to the first electrode portion (11) with respect to a center (164) of the second end face (160).

9. A corrosion measuring method comprising:
acquiring a current density (J) without contacting a test object (80) placed in water using a potential difference (V) between a pair of electrode portions (10); and
determining corrosion of the test object (80) using an acquired current density (J) or detecting corrosion of the test object (80) using the acquired current density (J).

10. The corrosion measuring method according to claim 9, wherein
the acquiring of the current density (J) includes:
measuring the potential difference (V) between the pair of electrode portions (10) without contacting the test object (80);
measuring a conductivity (σ) in the water; and
acquiring a current density (J) of a current (70, 71) flowing from the test object (80) into the water using a measured potential difference (V) and a measured conductivity (σ).

11. The corrosion measuring method according to claim 10, wherein
the measuring of the potential difference (V) includes measuring the potential difference (V) between the pair of electrode portions (10) without contacting a sacrificial anode (81) as the test object (80); and
the determining of corrosion of the test object (80) or the detecting of corrosion of the test object (80) includes determining corrosion of the sacrificial anode (81) using the acquired current density (J).

12. The corrosion measuring method according to claim 11, wherein the determining of corrosion of the sacrificial anode (81) includes estimating a sum of currents flowing from the sacrificial anode (81) by integrating acquired current densities and determining corrosion of the sacrificial anode (81) using an estimated sum of the currents.

13. The corrosion measuring method according to claim 10, wherein
the measuring of the potential difference (V) includes measuring the potential difference (V) between the pair of electrode portions (10) without contacting a non-corrosion-protected steel material (83) as the test object (80); and
the determining of corrosion of the test object (80) or the detecting of corrosion of the test object (80) includes detecting corrosion of the non-corrosion-protected steel material (83) using the acquired current density (J).

14. The corrosion measuring method according to claim 13, wherein the detecting of corrosion of the non-corrosion-protected steel material (83) includes detecting corrosion of the non-corrosion-protected steel material (83) using a peak portion (60) of a waveform of the acquired current density (J).
